# EUROPÄISCHE PATENTSCHRIFT

(11) **EP 4 622 583 B1**
(45) Veröffentlichungstag und Bekanntmachung des Hinweises auf die Patenterteilung: **07.10.2026**
(21) Anmeldenummer: 23805955.4
(22) Anmeldetag: 14.11.2023
(51) Int. Cl.: A61B 42/50, A47G 25/90

(54) **ANZIEHHILFE FÜR GUMMIHANDSCHUHE**
DONNING AID FOR RUBBER GLOVES
ACCESSOIRE POUR ENFILER DES GANTS EN CAOUTCHOUC

(30) Priorität: 21.11.2022 DE 102022130754
(43) Veröffentlichungstag der Anmeldung: 01.10.2025
(73) Patentinhaber: Friedrichs, Arno, 95326 Kulmbach (DE)
(72) Erfinder: Friedrichs, Arno, 95326 Kulmbach (DE)
(74) Vertreter: Zimmermann & Partner Patentanwälte mbB
(86) Internationale Anmeldenummer: PCT/EP2023/081728
(87) Internationale Veröffentlichungsnummer: WO 2024/110250

(56) Entgegenhaltungen:
- EP-B1- 3 253 262
- WO-A1-94/18900
- US-A- 3 695 493
- US-A- 4 915 272
- US-A- 5 058 785
- US-A- 5 078 308
- US-A1- 2007 062 970
- US-B1- 11 350 801
- US-B1- 6 193 117

## Beschreibung

Die Erfindung betrifft eine Anziehhilfe für Gummihandschuhe.

Aus der DE 20 2019 002 418 U1 ist eine Anziehhilfe für Handschuhe bekannt. Diese Anziehhilfe weist einen Griff, der eine längliche Ausdehnung hat und sich in einer Längsrichtung erstreckt, und mindestens drei nebeneinander angeordnete und voneinander beabstandete Fingerführungen auf, die an dem Griff angeordnet sind und den Griff in der Längsrichtung verlängern. Der Griff ist ausgestaltet, eine an den Griff angelegte und in Längsrichtung bewegte Hand eines Benutzers in Richtung der Fingerführungen entlang des Griffs zu führen, so dass ein Finger des Benutzers zu und entlang jeder der Fingerführungen geführt wird, um die Finger in einen auf die Vorrichtung aufgestülpten Fingerhandschuh zu führen.

Aus der DE 20 2021 101 702 U1 ist eine Anziehhilfe für insbesondere Kompressionshandschuhe bekannt, welche eine Schablone mit einem zumindest abschnittsweise hochgezogenen Rand und ein Halte-, Fixierungs- und/oder Verankerungselement zur zumindest temporären Fixierung oder Abstützung der Schablone beim Einführen der mit dem Handschuh zu bedeckenden Hand gegen elastische Rückstellkräfte des über die Schablone gezogenen und dabei entsprechend den Umrisskonturen der Schablone gedehnten Kompressionshandschuhs umfasst.

Aus der DE 197 19 808 A1 ist ein Handschuhspender mit Anziehhilfe bekannt. Dieser Handschuhspender dient einem berührungslosen Überstülpen von Gummihandschuhen vorwiegend im medizinischen Bereich. Der Handschuhspender hält den Handschuh in einer Vakuumkammer, die ein Vakuumgebläse aufweist, in einem aufgeblähten Zustand bereit. Die Hand des Benutzers wird in den geblähten Handschuh gesteckt und dann weiter durchgeschoben, so dass der Handschuh von einem Köcher gerissen wird und durch ein Brechen des Vakuums an die Hand des Benutzers gelegt wird.

Aus der US 2007/062970 A1 ist eine Vorrichtung zum Ausgeben eines Gegenstands, insbesondere eines Handschuhs, mit einem Körperteil und einer Öffnung, die den Zugang zum Inneren des Körperteils ermöglicht, bekannt. Diese Vorrichtung umfasst eine Kammer mit einer Mündung, die so geformt und dimensioniert ist, dass sie die Öffnung des Gegenstandes aufnimmt, um eine im Wesentlichen flüssigkeitsdichte Abdichtung an der Öffnung zu schaffen, so dass sich zumindest ein Teil des oder jedes Körperteils im Inneren der Kammer befindet. Die Vorrichtung umfasst ferner Mittel zur Erzeugung eines Druckunterschieds zwischen der Innenfläche und der Außenfläche des mindestens einen Gegenstands. Der Druck bewirkt, dass sich der Gegenstand nach außen ausdehnt, was das Einführen einer Hand oder eines anderen Gegenstands erleichtert.

Weitere Anziehhilfen für Gummihandschuhe sind aus den Druckschriften US 11 350 801 B1, WO 94/18900 A1, US 3 695 493 A, US 4 915 272 A, US 5 058 785 A, EP 3 253 262 B1, US 5 078 308 A und US 6 193 117 B1 bekannt.

Ausgehend von diesem Stand der Technik liegt der Erfindung die Aufgabe zugrunde, eine Anziehhilfe für Gummihandschuhe anzugeben, die ein Anziehen eines Gummihandschuhs in einem evakuierbaren Behälter ermöglicht, ohne dass es einer Entnahme des anzuziehenden Gummihandschuhs aus einem innerhalb des evakuierbaren Behälters angeordneten Magazin bedarf.

Diese Aufgabe wird durch eine Anziehhilfe mit den im Anspruch 1 angegebenen Merkmalen gelöst. Vorteilhafte Ausgestaltungen und Weiterbildungen der Erfindung sind in den abhängigen Ansprüchen angegeben.

Eine erfindungsgemäße Anziehhilfe für Gummihandschuhe weist einen evakuierbaren Behälter, der eine Öffnung aufweist, ein ringförmiges Formteil, welches zusammen mit dem Gummihandschuh zum luftdichten Verschließen der Öffnung des evakuierbaren Behälters verbunden ist, und Mittel zur Evakuierung des luftdicht verschlossenen Behälters auf, wobei die Innenwand des evakuierbaren Behälters mit einer auswechselbaren Folie ausgestattet ist.

Der umlaufende Rand dieses Kunststoffringes kann kreisförmig oder oval ausgeführt sein.

Gemäß einer Ausführungsform handelt es sich bei dem ringförmigen Formteil um einen Kunststoffring. Gemäß einer weiteren Ausführungsform handelt es sich bei dem ringförmigen Formteil um einen Ring aus einem recyclebaren Material.

Gemäß einer Ausführungsform ist der evakuierbare Behälter hohlzylindrisch ausgebildet. Dies verbessert die Handhabbarkeit der Anziehhilfe beim Anziehen des Gummihandschuhs und gewährleistet eine hohe Stabilität des evakuierbaren Behälters beim Auftreten von Druckveränderungen innerhalb des evakuierbaren Behälters.

Gemäß einer Ausführungsform ist die Öffnung des evakuierbaren Behälters an einer Stirnseite des evakuierbaren Behälter positioniert. Dies erleichtert das luftdichte Verschließen der Öffnung des evakuierbaren Behälters.

Gemäß einer Ausführungsform ist das ringförmige Formteil in dem von den Fingern des Gummihandschuhs abgelegenen Endbereich des Gummihandschuhs mit dem Gummihandschuh verbunden. Dies erleichtert ein Auflegen des Gummihandschuhs auf die Öffnung des evakuierbaren Behälters derart, dass nach diesem Auflegen des ringförmigen Formteils auf die Öffnung des evakuierbaren Behälters die Finger des Gummihandschuhs innerhalb des evakuierbaren Behälters positioniert sind.

Gemäß einer Ausführungsform ist das ringförmige Formteil in dem von den Fingern des Gummihandschuhs abgelegenen Endbereich des Gummihandschuhs mit dem Gummihandschuh fest verbunden. Dies erleichtert das luftdichte Verschließen der Öffnung des evakuierbaren Behälters.

Gemäß einer Ausführungsform ist das ringförmige Formteil in dem von den Fingern des Gummihandschuhs abgelegenen Endbereich des Gummihandschuhs in den Gummihandschuh eingearbeitet. Auch dadurch wird das luftdichte Verschließen der Öffnung des evakuierbaren Behälters verbessert.

Gemäß einer Ausführungsform ist zum luftdichten Verschließen der Öffnung des evakuierbaren Behälters ein parallel zum ringförmigen Formteil angeordnetes weitere ringförmiges Formteil vorgesehen, wobei der von den Fingern des Gummihandschuhs abgelegene Endbereich des Gummihandschuhs zwischen den beiden Formteilen eingeklemmt ist. Dies verbessert die zum luftdichten Verschließen der Öffnung des evakuierbaren Behälters notwendige feste Verbindung zwischen dem Gummihandschuh und dem ringförmigen Formteil.

Gemäß einer Ausführungsform sind die Mittel zur Evakuierung des luftdicht verschlossenen Behälters in Form eines Faltenbalges realisiert. Mittels eines derartigen Faltenbalges können in mechanisch einfacher Weise Druckveränderungen im Innenbereich des evakuierbaren Behälters durchgeführt werden.

Gemäß einer Ausführungsform ist der Faltenbalg in dem von der Öffnung des evakuierbaren Behälters abgelegenen Endbereich des evakuierbaren Behälters angeordnet. Dies erleichtert die Durchführung von Druckveränderungen innerhalb des evakuierbaren Behälters.

Gemäß einer Ausführungsform ist der Faltenbalg manuell betätigbar.

Gemäß einer Ausführungsform ist der Faltenbalg im evakuierbaren Behälter federgelagert ist. Dies erleichtert die manuelle Betätigung des Faltenbalges.

Gemäß einer Ausführungsform sind die Mittel zur Evakuierung des luftdicht verschlossenen Behälters in Form eines Motors realisiert.

Gemäß einer Ausführungsform ist innerhalb des evakuierbaren Behälters ein Drucksensor positioniert. Dies ermöglicht eine bedarfsgerechte Ansteuerung des Motors zur gewünschten Evakuierung des luftdicht verschlossenen Behälters.

Die vorstehend beschriebenen Ausführungsformen können gemeinsam verwendet werden, sofern sie sich nicht gegenseitig ausschließen.

Nachfolgend werden Ausführungsbeispiele für die Erfindung anhand der Zeichnungen näher erläutert. Es zeigt
- Figur 1 ein erstes Ausführungsbeispiel zur Veranschaulichung einer Anziehhilfe für Gummihandschuhe gemäß der Erfindung und
- Figur 2 ein zweites Ausführungsbeispiel zur Veranschaulichung einer Anziehhilfe für Gummihandschuhe gemäß der Erfindung.

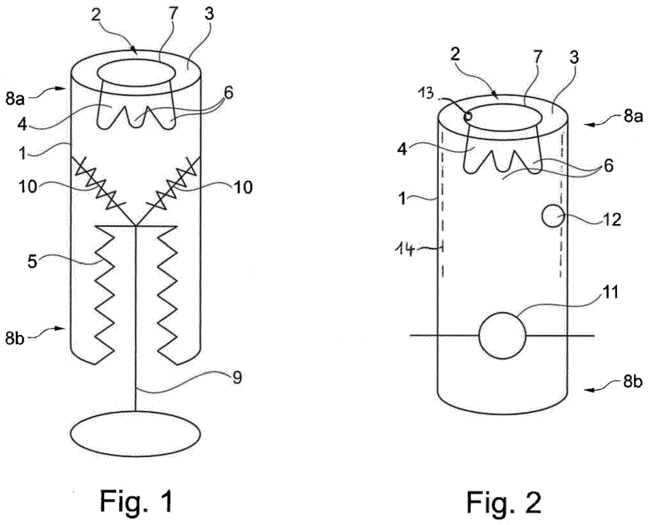

Die Figur 1 zeigt ein erstes Ausführungsbeispiel zur Veranschaulichung einer Anziehhilfe für Gummihandschuhe gemäß der Erfindung.

Die in der Figur 1 gezeigte Anziehhilfe für Gummihandschuhe weist einen evakuierbaren Behälter 1, der eine Öffnung 2 aufweist, einen Kunststoffring 3, der mit einem Gummihandschuh 4 zum luftdichten Verschließen der Öffnung des evakuierbaren Behälters verbunden ist, und Mittel 5 zur Evakuierung des luftdicht verschlossenen Behälters auf. Bei dem Kunststoffring handelt es sich um einen Kunststoffring, dessen umlaufender Rand kreisförmig ausgebildet ist, oder um einen Kunststoffring, dessen umlaufender Rand oval ausgebildet ist.

Der evakuierbare Behälter 1 ist hohlzylindrisch ausgebildet. Seine Öffnung 2 ist an einer Stirnseite des evakuierbaren Behälters 1 positioniert. Der Kunststoffring 3 ist in dem von den Fingern 6 des Gummihandschuhs 4 abgelegenen Endbereich des Gummihandschuhs mit dem Gummihandschuh verbunden.

Bei dieser Verbindung des Kunststoffrings 3 mit dem Gummihandschuh 4 handelt es sich um eine feste Verbindung, die ein luftdichtes Verschließen der Öffnung 2 des evakuierbaren Behälters 1 ermöglicht, beispielsweise eine Klebeverbindung.

Alternativ dazu kann die genannte feste Verbindung auch dadurch realisiert sein, dass der Kunststoffring 3 in dem von den Fingern 6 des Gummihandschuhs 4 abgelegenen Endbereich des Gummihandschuhs in den Gummihandschuh eingearbeitet ist. Beispielsweise ist der Kunststoffring 3 in dem von den Fingern 6 des Gummihandschuhs abgelegenen Endbereich des Gummihandschuhs von Gummimaterial des Gummihandschuhs vollständig umgeben.

Eine weitere Alternative zur Realisierung einer festen Verbindung des Kunststoffrings mit dem Gummihandschuh besteht darin, parallel zum Kunststoffring 3 einen weiteren Kunststoffring derart vorzusehen, dass der von den Fingern des Gummihandschuhs abgelegene Endbereich des Gummihandschuhs zwischen den beiden Kunststoffringen eingeklemmt ist.

Die Mittel zur Evakuierung des luftdicht verschlossenen Behälters 1 sind bei dem in der Figur 1 gezeigten Ausführungsbeispiel in Form eines Faltenbalges 5 realisiert. Dieser Faltenbalg 5 ist in dem von der Öffnung 2 des evakuierbaren Behälters 1 abgelegenen Endbereich 8b des evakuierbaren Behälters 1 angeordnet. Dieser Faltenbalg 5 ist federgelagert. Seine obere Begrenzungswand ist mittels zweier Federn 10, die jeweils schräg nach oben führen, am Innenmantel des hohlzylindrisch ausgebildeten Behälters 1 befestigt. Des Weiteren ist auf der Unterseite der oberen Begrenzungswand des Faltenbalges 5 ein nach unten führendes Betätigungselement 9 befestigt, welches in seinem unteren Endbereich eine Griffschleife bildet. Durch ein Ziehen des Betätigungselements 9 an seiner Griffschleife nach unten wird die obere Begrenzungswand des Faltenbalges 5 nach unten gezogen. Dadurch wird der im Innenbereich des Behälters herrschende Luftdruck zunehmend reduziert, sofern die im oberen Endbereich 8a des Behälters 1 vorgesehene Öffnung 2 luftdicht verschlossen ist.

Zum Anziehen eines Gummihandschuhs wird der hohlzylindrische Behälter 1 in einem ersten Schritt durch ein Auflegen des mit dem Gummihandschuh 4 verbundenen Kunststoffrings 3 auf die Öffnung 2 des Behälters 1 luftdicht verschlossen, wobei die Finger 6 des Gummihandschuhs innerhalb des hohlzylindrischen Behälters positioniert werden.

In einem nachfolgenden zweiten Schritt erfolgt durch ein Herunterziehen der oberen Begrenzungswand des Faltenbalges 5 durch eine fortlaufende Reduzierung des im Behälter 1 herrschenden Luftdrucks eine Evakuierung des Innenraums des hohlzylindrischen Behälters. Durch diese Evakuierung des Innenraums des hohlzylindrischen Behälters wird der Gummihandschuh in seinem innerhalb des hohlzylindrischen Behälters liegenden Bereich nach unten gezogen und aufgebläht, so dass der Person, die den Gummihandschuh anziehen möchte, der Gummihandschuh in seinem aufgeblähten Zustand zum Anziehen bereitgestellt wird. Nach dem Anziehen des Gummihandschuhs wird der evakuierte Innenraum des Behälters 1 durch ein Herausziehen der mit dem angezogenen Gummihandschuh ausgestatteten Hand der genannten Person wieder belüftet, so dass sich der Gummihandschuh eng an die Hand dieser Person anlegt.

Die Figur 2 zeigt ein zweites Ausführungsbeispiel zur Veranschaulichung einer Anziehhilfe für Gummihandschuhe gemäß der Erfindung.

Die in der Figur 2 gezeigte Anziehhilfe für Gummihandschuhe weist einen evakuierbaren Behälter 1, der eine Öffnung 2 aufweist, einen Kunststoffring 3, der mit einem Gummihandschuh 4 zum luftdichten Verschließen der Öffnung des evakuierbaren Behälters verbunden ist, und Mittel 11 zur Evakuierung des luftdicht verschlossenen Behälters auf.

Der evakuierbare Behälter 1 ist hohlzylindrisch ausgebildet. Seine Öffnung 2 ist an einer Stirnseite des evakuierbaren Behälters 1 positioniert. Der Kunststoffring 3 ist in dem von den Fingern 6 des Gummihandschuhs 4 abgelegenen Endbereich des Gummihandschuhs mit dem Gummihandschuh verbunden.

Bei dieser Verbindung des Kunststoffrings 3 mit dem Gummihandschuh 4 handelt es sich um eine feste Verbindung, die ein luftdichtes Verschließen der Öffnung 2 des evakuierbaren Behälters 1 ermöglicht, beispielsweise eine Klebeverbindung.

Alternativ dazu kann die genannte feste Verbindung auch dadurch realisiert sein, dass der Kunststoffring 3 in dem von den Fingern 6 des Gummihandschuhs 4 abgelegenen Endbereich des Gummihandschuhs in den Gummihandschuh eingearbeitet ist. Beispielsweise ist der Kunststoffring 3 in dem von den Fingern 6 des Gummihandschuhs abgelegenen Endbereich des Gummihandschuhs von Gummimaterial des Gummihandschuhs allseitig umgeben.

Eine weitere Alternative zur Realisierung einer festen Verbindung des Kunststoffrings mit dem Gummihandschuh besteht darin, parallel zum Kunststoffring 3 einen weiteren Kunststoffring derart vorzusehen, dass der von den Fingern des Gummihandschuhs abgelegene Endbereich des Gummihandschuhs zwischen den beiden Kunststoffringen eingeklemmt ist.

Die Mittel zur Evakuierung des luftdicht verschlossenen Behälters 1 sind bei dem in der Figur 2 gezeigten Ausführungsbeispiel in Form eines Motors 11 realisiert. Dieser Motor 11 ist in dem von der Öffnung 2 des evakuierbaren Behälters 1 abgelegenen Endbereich 8b des evakuierbaren Behälters 1 angeordnet. Dieser Motor 11 wird zur Bewegung eines in der Figur 2 nicht dargestellten Kolbens verwendet, wobei durch diese Bewegung des Kolbens der im Innenbereich des hohlzylindrisch ausgebildeten Behälters herrschende Druck veränderbar ist. Beispielsweise wird der im Innenbereich des Behälters herrschende Luftdruck zunehmend reduziert, sofern die im oberen Endbereich 8a des Behälters 1 vorgesehene Öffnung 2 luftdicht verschlossen ist. Die notwendige Steuerung des Motors 11 erfolgt unter Berücksichtigung der Ausgangssignale eines innerhalb des Behälters 2 angeordneten Drucksensors 12.

Zum Anziehen eines Gummihandschuhs wird der hohlzylindrische Behälter 1 in einem ersten Schritt durch ein Auflegen des mit dem Gummihandschuh verbundenen Kunststoffrings 3 auf die Öffnung 2 des Behälters 1 luftdicht verschlossen, wobei die Finger 6 des Gummihandschuhs innerhalb des hohlzylindrischen Behälters positioniert werden.

In einem nachfolgenden zweiten Schritt erfolgt durch eine von den Ausgangssignalen des Drucksensors 12 gesteuerte Aktivierung des Motors 11 eine fortlaufende Reduzierung des im Behälter 1 herrschenden Luftdrucks solange, bis die gewünschte Evakuierung des Innenraums des hohlzylindrischen Behälters erreicht ist. Durch diese Evakuierung des Innenraums des hohlzylindrischen Behälters wird der Gummihandschuh in seinem innerhalb des hohlzylindrischen Behälters liegenden Bereich nach unten gezogen und aufgebläht, so dass der Person, die den Gummihandschuh anziehen möchte, der Gummihandschuh in seinem aufgeblähten Zustand zum Anziehen bereitgestellt wird. Nach dem Anziehen des Gummihandschuhs wird der evakuierte Innenraum des Behälters 1 durch ein Herausziehen der mit dem angezogenen Gummihandschuh ausgestatteten Hand der genannten Person wieder belüftet, so dass sich der Gummihandschuh eng an die Hand dieser Person anlegt. Alternativ oder zusätzlich dazu kann die Wiederbelüftung des Innenraums des Behälters 2 auch mittels des Motors 11 vorgenommen werden.

Bei beiden vorstehend beschriebenen Ausführungsbeispielen ist der Innendurchmesser des Kunststoffrings 3 derart gewählt, dass die Hand einer Person, die den Gummihandschuh anziehen möchte, zum Anziehen des Gummihandschuhs durch den Kunststoffring geschoben werden kann.

Bei beiden vorstehend beschriebenen Ausführungsbeispielen kann die Anziehhilfe auch zum Ausziehen eines jeweiligen Gummihandschuhs verwendet werden.

Eine vorteilhafte Ausgestaltung der Erfindung besteht darin, zum Erreichen bzw. Sicherstellen einer gewünschten Positionierung des mit dem Kunststoffring verbundenen Gummihandschuhs auf dem evakuierbaren Behälter einen Magneten 13 zur Hilfe zu nehmen. Dies ist insbesondere von Vorteil, wenn der umlaufende Rand des Kunststoffringes oval ausgebildet ist. Dieser Magnet 13 ist beispielsweise am oder im ringförmigen Formteil positioniert und arbeitet mit einem zweiten Magneten oder einem aus Metall bestehenden Gegenstück zusammen, welches auf einem verbreiterten oberen Rand des evakuierbaren Behälters angeordnet ist. Alternativ dazu kann das aus Metall bestehende Gegenstück am oder im ringförmigen Formteil positioniert sein und der Magnet 13 auf oder im Gegenstück vorgesehen sein.

Eine weitere vorteilhafte Ausgestaltung der Erfindung besteht darin, das ringförmige Formteil als Positionierungshilfe mit einer nasenförmigen Ausbuchtung zu versehen, die mit einer zugehörigen Einkerbung auf dem verbreiterten oberen Rand des evakuierbaren Behälters zusammenwirkt.

Eine weitere vorteilhafte Ausgestaltung der Erfindung besteht darin, das ringförmige Formteil mit einer leichten Wellenform zu versehen.

Erfindungsgemäß ist die Innenwand des evakuierbaren Behälters aus hygienischen Gründen mit einer auswechselbaren Folie 14 ausgestattet.

Diese Folie 14 kann den gesamten Umfangsbereich des evakuierbaren Behälters abdecken und unten offen ausgebildet sein. Alternativ dazu kann die Folie 14 auch in Vertikalrichtung kürzer ausgebildet sein und lediglich den Umfangsbereich des evakuierbaren Behälters abdecken, beim Anziehen des Gummihandschuhs vom Gummihandschuh berührt wird.

### Bezugszeichenliste

- 1: evakuierbarer Behälter
- 2: Öffnung des evakuierbaren Behälters
- 3: ringförmiges Formteil; Kunststoffring
- 4: Gummihandschuh
- 5: Faltenbalg
- 6: Finger des Gummihandschuhs
- 7: Verbindungsbereich
- 8a: oberer Endbereich des evakuierbaren Behälters
- 8b: unterer Endbereich des evakuierbaren Behälters
- 9: Betätigungselement
- 10: Feder
- 11: Motor
- 12: Drucksensor
- 13: Magnet
- 14: Folie (Hygienefolie)

## Patentansprüche

1. Anziehhilfe für Gummihandschuhe, welche aufweist:
- einen evakuierbaren Behälter (1), der eine Öffnung (2) aufweist,
- ein ringförmiges Formteil (3), welches mit dem Gummihandschuh (4) zum luftdichten Verschließen der Öffnung (2) des evakuierbaren Behälters (1) verbunden ist, und
- Mittel (5, 11) zur Evakuierung des luftdicht verschlossenen Behälters, **dadurch gekennzeichnet, dass** die Innenwand des evakuierbaren Behälters mit einer auswechselbaren Folie (14) ausgestattet ist.

2. Anziehhilfe nach Anspruch 1, bei welcher der evakuierbare Behälter (1) hohlzylindrisch ausgebildet ist.

3. Anziehhilfe nach Anspruch 2, bei welcher die Öffnung (2) des evakuierbaren Behälters an einer Stirnseite des evakuierbaren Behälters (1) positioniert ist.

4. Anziehhilfe nach einem der vorhergehenden Ansprüche, bei welcher das ringförmige Formteil (3) in dem von den Fingern (6) des Gummihandschuhs abgelegenen Endbereich des Gummihandschuhs mit dem Gummihandschuh verbunden ist.

5. Anziehhilfe nach Anspruch 4, bei welcher das ringförmige Formteil (3) in dem von den Fingern (6) des Gummihandschuhs abgelegenen Endbereich des Gummihandschuhs mit dem Gummihandschuh fest verbunden ist.

6. Anziehhilfe nach Anspruch 4, bei welcher das ringförmige Formteil (3) in dem von den Fingern (6) des Gummihandschuhs abgelegenen Endbereich des Gummihandschuhs in den Gummihandschuh eingearbeitet ist.

7. Anziehhilfe nach Anspruch 4, bei welchem zum luftdichten Verschließen der Öffnung (2) des evakuierbaren Behälters (1) ein parallel zum ringförmigen Formteil angeordnetes weiteres ringförmiges Formteil vorgesehen ist, wobei der von den Fingern des Gummihandschuhs abgelegene Endbereich des Gummihandschuhs zwischen den beiden ringförmigen Formteilen eingeklemmt ist.

8. Anziehhilfe nach einem der vorhergehenden Ansprüche, bei welchem die Mittel zur Evakuierung des luftdicht verschlossenen Behälters in Form eines Faltenbalges (5) realisiert sind.

9. Anziehhilfe nach Anspruch 8, bei welcher der Faltenbalg (5) in dem von der Öffnung des evakuierbaren Behälter abgelegenen Endbereich des evakuierbaren Behälters angeordnet ist.

10. Anziehhilfe nach Anspruch 8 oder 9, bei welcher der Faltenbalg (5) manuell betätigbar ist.

11. Anziehhilfe nach einem der Ansprüche 8 - 10, bei welcher der Faltenbalg (5) im evakuierbaren Behälter federgelagert ist.

12. Anziehhilfe nach einem der Ansprüche 1 - 7, bei welcher die Mittel zur Evakuierung des luftdicht verschlossenen Behälters in Form eines Motors (11) realisiert sind.

13. Anziehhilfe nach Anspruch 12, bei welcher innerhalb des evakuierbaren Behälters ein Drucksensor (12) positioniert ist.

14. Anziehhilfe nach einem der vorhergehenden Ansprüche, welche einen Magneten (13) aufweist, der zur Positionierung des mit dem ringförmigen Formteil verbundenen Gummihandschuhs auf dem evakuierbaren Behälter ausgebildet ist.

15. Anziehhilfe nach einem der vorhergehenden Ansprüche, bei welcher das ringförmige Formteil (3) ein Kunststoffring ist.

16. Anziehhilfe nach einem der vorhergehenden Ansprüche 1 - 15, bei welchem das ringförmige Formteil (3) aus einem recyclebaren Material besteht.

## Claims

1. Donning aid for rubber gloves, which comprises:
- an evacuable container (1) having an opening (2),
- an annular molded part (3), which is connected to the rubber glove (4) for the airtight sealing of the opening (2) of the evacuable container (1), and
- means (5, 11) for evacuating the airtightly sealed container,
**characterized in that**
the inner wall of the evacuable container is equipped with a replaceable film (14).

2. Donning aid according to claim 1, in which the evacuable container (1) is formed as a hollow cylinder.

3. Donning aid according to claim 2, in which the opening (2) of the evacuable container is positioned on an end face of the evacuable container (1).

4. Donning aid according to any one of the preceding claims, in which the annular molded part (3) is connected to the rubber glove in the end region of the rubber glove remote from the fingers (6) of the rubber glove.

5. Donning aid according to claim 4, in which the annular molded part (3) is firmly connected to the rubber glove in the end region of the rubber glove remote from the fingers (6) of the rubber glove.

6. Donning aid according to claim 4, in which the annular molded part (3) is incorporated into the rubber glove in the end region of the rubber glove remote from the fingers (6) of the rubber glove.

7. Donning aid according to claim 4, in which, for the airtight sealing of the opening (2) of the evacuable container (1), a further annular molded part arranged parallel to the annular molded part is provided, wherein the end region of the rubber glove remote from the fingers of the rubber glove is clamped between the two annular molded parts.

8. Donning aid according to any one of the preceding claims, in which the means for evacuating the airtightly sealed container are realized in the form of a bellows (5).

9. Donning aid according to claim 8, in which the bellows (5) is arranged in the end region of the evacuable container remote from the opening of the evacuable container.

10. Donning aid according to claim 8 or 9, in which the bellows (5) can be actuated manually.

11. Donning aid according to any one of claims 8 to 10, in which the bellows (5) is spring-mounted in the evacuable container.

12. Donning aid according to any one of claims 1 to 7, in which the means for evacuating the airtightly sealed container are realized in the form of a motor (11).

13. Donning aid according to claim 12, in which a pressure sensor (12) is positioned inside the evacuable container.

14. Donning aid according to any one of the preceding claims, which has a magnet (13) configured for positioning the rubber glove connected to the annular molded part on the evacuable container.

15. Donning aid according to any one of the preceding claims, in which the annular molded part (3) is a plastic ring.

16. Donning aid according to any one of the preceding claims 1 to 15, in which the annular molded part (3) consists of a recyclable material.

## Revendications

1. Aide à l'enfilage pour des gants en caoutchouc, qui présente :
- un récipient (1) dans lequel le vide peut être fait, qui présente une ouverture (2),
- une pièce moulée annulaire (3), qui est reliée au gant en caoutchouc (4) pour fermer hermétiquement l'ouverture (2) du récipient (1) dans lequel le vide peut être fait, et
- des moyens (5, 11) pour faire le vide dans le récipient fermé hermétiquement, **caractérisée en ce que**
la paroi intérieure du récipient dans lequel le vide peut être fait est équipée d'un film interchangeable (14).

2. Aide à l'enfilage selon la revendication 1, dans laquelle le récipient (1) dans lequel le vide peut être fait est réalisé de manière cylindrique creuse.

3. Aide à l'enfilage selon la revendication 2, dans laquelle l'ouverture (2) du récipient dans lequel le vide peut être fait est positionnée sur une face frontale du récipient (1) dans lequel le vide peut être fait.

4. Aide à l'enfilage selon l'une des revendications précédentes, dans laquelle la pièce moulée annulaire (3) est reliée au gant en caoutchouc dans la zone d'extrémité du gant en caoutchouc éloignée des doigts (6) du gant en caoutchouc.

5. Aide à l'enfilage selon la revendication 4, dans laquelle la pièce moulée annulaire (3) est solidaire du gant en caoutchouc dans la zone d'extrémité du gant en caoutchouc éloignée des doigts (6) du gant en caoutchouc.

6. Aide à l'enfilage selon la revendication 4, dans laquelle la pièce moulée annulaire (3) est intégrée au gant en caoutchouc dans la zone d'extrémité du gant en caoutchouc éloignée des doigts (6) du gant en caoutchouc.

7. Aide à l'enfilage selon la revendication 4, dans laquelle une autre pièce moulée annulaire, disposée parallèlement à la pièce moulée annulaire, est prévue pour fermer hermétiquement l'ouverture (2) du récipient (1) dans lequel le vide peut être fait, dans laquelle la zone d'extrémité du gant en caoutchouc éloignée des doigts du gant en caoutchouc est coincée entre les deux pièces moulées annulaires.

8. Aide à l'enfilage selon l'une des revendications précédentes, dans laquelle les moyens pour faire le vide dans le récipient fermé hermétiquement sont réalisés sous la forme d'un soufflet (5).

9. Aide à l'enfilage selon la revendication 8, dans laquelle le soufflet (5) est disposé dans la zone d'extrémité du récipient dans lequel le vide peut être fait éloignée de l'ouverture du récipient dans lequel le vide peut être fait.

10. Aide à l'enfilage selon la revendication 8 ou 9, dans laquelle le soufflet (5) peut être actionné manuellement.

11. Aide à l'enfilage selon l'une des revendications 8-10, dans laquelle le soufflet (5) est monté sur ressort dans le récipient dans lequel le vide peut être fait.

12. Aide à l'enfilage selon l'une des revendications 1-7, dans laquelle les moyens pour faire le vide dans le récipient fermé hermétiquement sont réalisés sous la forme d'un moteur (11).

13. Aide à l'enfilage selon la revendication 12, dans laquelle un capteur de pression (12) est positionné à l'intérieur du récipient dans lequel le vide peut être fait.

14. Aide à l'enfilage selon l'une des revendications précédentes, qui présente un aimant (13) qui est conçu pour positionner le gant en caoutchouc relié à la pièce moulée annulaire sur le récipient dans lequel le vide peut être fait.

15. Aide à l'enfilage selon l'une des revendications précédentes, dans laquelle la pièce moulée annulaire (3) est une bague en plastique.

16. Aide à l'enfilage selon l'une des revendications précédentes 1 à 15, dans laquelle la pièce moulée annulaire (3) est constituée d'un matériau recyclable.
